# EUROPÄISCHE PATENTSCHRIFT

(11) **EP 1 599 434 B1**
(45) Veröffentlichungstag und Bekanntmachung des Hinweises auf die Patenterteilung: **12.12.2007**
(21) Anmeldenummer: 04710347.8
(22) Anmeldetag: 12.02.2004
(51) Int. Cl.: C07C 31/13, C11B 9/00

(54) **4-CYCLOHEXYL-2-BUTANOL ALS RIECHSTOFF**
4-CYCLOHEXYL-2-BUTANOL AS AN ODIFEROUS SUBSTANCE
4-CYCLOHEXYL-2-BUTANOL SERVANT DE PARFUM

(30) Priorität: 26.02.2003 DE 10308047
(43) Veröffentlichungstag der Anmeldung: 30.11.2005
(73) Patentinhaber: Symrise GmbH & Co. KG, 37603 Holzminden (DE)
(72) Erfinder: KUHN, Walter, 37603 Holzminden (DE); BORK, Karl-Heinz, 37627 Deensen (DE); SURBURG, Horst, 37603 Holzminden (DE)
(74) Vertreter: Eisenführ, Speiser & Partner
(86) Internationale Anmeldenummer: PCT/EP2004/001282
(87) Internationale Veröffentlichungsnummer: WO 2004/076393

(56) Entgegenhaltungen:
- EP-A- 0 180 885
- US-A- 2 447 050

## Beschreibung

Die Erfindung betrifft primär die Verwendung von 4-Cyclohexyl-2-butanol als Riechstoff und zwar insbesondere als Maiglöckchen-Riechstoff. Weiterhin betrifft die Erfindung Mischungen von Riechstoffen und parfümierte Produkte, die jeweils 4-Cyclohexyl-2-butanol enthalten, sowie ein Verfahren zur Herstellung eines entsprechenden parfümierten Produktes.

Riechstoffe mit Maiglöckchengeruch werden in großer Menge in Parfüms, Riechstoffmischungen (Parfümkompositionen) und Parfümierungen für die verschiedensten Anwendungsgebiete eingesetzt. Maiglöckchenriechstoffe verleihen Riechstoffkompositionen durch ihren maiglöckchenartigen Geruch eine saubere, klare, transparente Frische, die von anderen Blütenriechstoffen nicht erreicht wird. Maiglöckchengeruch wird im Vergleich mit sonstigen Gerüchen am eindeutigsten mit dem Empfinden von Frische und Sauberkeit assoziiert, und deshalb sind Maiglöckchenriechstoffe unter den Blütenriechstoffen von besonderem Interesse. Dementsprechend hoch ist der von der Riechstoffindustrie betriebene Aufwand bei der Ermittlung neuer industrie betriebene Aufwand bei der Ermittlung neuer Riechstoffe mit Maiglöckchengeruch.

Die Suche nach geeigneten Riechstoffen mit Maiglöckchengeruch wird für den Fachmann aber insbesondere durch folgende Sachverhalte erschwert:
- Die Mechanismen der Geruchswahrnehmung sind nicht ausreichend bekannt.
- Eine objektiv-quantitative Charakterisierung eines Geruches ist derzeit noch nicht möglich.
- Die Zusammenhänge zwischen der speziellen Geruchswahrnehmung einerseits und der chemischen Struktur des zugehörigen Riechstoffs andererseits sind nicht hinreichend erforscht.
- Häufig bewirken bereits geringfügige Änderungen am strukturellen Aufbau eines bekannten Riechstoffs starke Änderungen der olfaktorischen Eigenschaften und beeinträchtigen die Verträglichkeit für den menschlichen Organismus.

Der Erfolg der Suche nach geeigneten Riechstoffen (im vorliegenden Fall mit Maiglöckchengeruch) hängt deshalb stark von der Intuition des Suchenden ab.

Die kommerziell bedeutendsten Maiglöckchen-Riechstoffe sind derzeit 3-(4t-Butylphenyl)-2-methylpropanal (Handelsnamen z.B. Lilial, Lysmeral, Lilestralis), 3,7-Dimethyl-7-hydroxyoctanal (Trivialname Hydroxycitronellal) und 4-(4-Hydroxy-4-methylpentyl)-3-cyclohexencarboxaldehyd (Handelsnamen z.B. Kovanol, Lyral).

Viele Menschen reagieren jedoch auf diese Stoffe mit Unverträglichkeiten wie z.B. mit allergischen Reaktionen. Es besteht daher Bedarf an alternativen Riechstoffen mit Maiglöckchengeruch, insbesondere solchen, die die oben genannten Verbindungen in Parfümkompositionen zu ersetzen vermögen und die keine derartigen negativen toxikologischen Eigenschaften aufweisen. Von erheblichem Vorteil ist es dabei, wenn diese Alternativprodukte sowohl in ihren sensorisch-geruchlichen als auch in ihren anwendungstechnischen Eigenschaften den zu ersetzenden Riechstoffen möglichst ähnlich sind.

Es wurde nun gefunden, dass 4-Cyclohexyl-2-butanol ein hervorragender Riechstoff mit einem besonders natürlichen und reintönigen Maiglöckchengeruch ist, der in Parfümkompositionen die bislang eingesetzten (allergieauslösenden) Riechstoffe mit Maiglöckchengeruch ersetzen kann.

Dieses Ergebnis ist insbesondere deshalb überraschend, weil als Riechstoffe bekannte Substanzen mit Strukturen, die der des 4-Cyclohexyl-2-butanol ähneln und die teilweise ebenfalls maiglöckchenartige Geruchsnoten aufweisen, den gewünschten Effekt nicht erbringen können. Solche Stoffe sind z.B. 2-Methyl-1-phenyl-2-propanol, 2-Methyl-4-phenyl-2-butanol, 3-Methyl-1-phenyl-3-pentanol, 5-Phenyl-3-pentanol (z.B. Steffen Arctander, Perfume and Flavor Chemicals, Eigenverlag, Montclair, N.J., 1969; K. Bauer, D. Garbe, H. Surburg, Common Fragrance and Flavor Materials, 4th Edition, Wiley-VCH, Weinheim 2001), 1-(4-Isopropylcyclohexyl)ethanol (R. Pelzer et al. in R. Hopp and K. Mori, Recent Developments in Flavor and Fragrance Chemistry, VCH, Weinheim 1993, S. 29 ff), 1-Cyclohexyl-2-methyl-2-propanol (EP 1108703), 4-Cyclohexyl-3-methyl-2-butanol (US 5372995) und 4-Cyclohexyl-2-methyl-2-butanol (EP 180885). Alle diese Stoffe zeigen nicht so einen natürlichen und reintönigen Maiglöckchenduft wie das erfindungsgemäße 4-Cyclohexyl-2-butanol. Dazu im Einzelnen:
- 2-Methyl-1-phenyl-2-propanol riecht krautig-blumig, eher nach Flieder.
- Der Geruch von 2-Methyl-4-phenyl-2-butanol ist blumig, aber etwas grün und krautig, eher in die Richtung von Hyacinthen und Lilien gehend.
- 3-Methyl-1-phenyl-3-pentanol zeigt zwar einen untergeordneten Maiglöckchengeruch, dominant ist aber eine an Päoniengeruch erinnernde Rosennote.
- 5-Phenyl-3-pentanol hat einen blumig grünen Geruch, der nur wenig an Maiglöckchen erinnert.
- 1-(4-Isopropylcyclohexyl)ethanol riecht zwar vorwiegend nach Maiglöckchen, weist jedoch wegen einer leicht würzigen, grün-erdigen fliederartigen Note weitem nicht so einen reintönigen und charakteristischen Maiglöckchengeruch wie das erfindungsgemäße 4-Cyclohexyl-2-butanol auf.
- Der Geruch von 1-Cyclohexyl-2-methyl-2-propanol wird als frisch, blumig, fliederartig und minzig beschrieben; diese Verbindung ist daher als Maiglöckchen-Riechstoff weniger geeignet.
- 4-Cyclohexyl-3-methyl-2-butanol und 4-Cyclohexyl-2-methyl-2-butanol riechen weniger nach Maiglöckchen als vielmehr nach Linalool (3,7-Dimethyl-1,6-octadien-3-ol) und Dihydromyrcenol (2,6-Dimethyl-7-octen-2-ol), die einen eher frisch-blumigen citrusartigen Geruchscharakter haben (Steffen Arctander, Perfume and Flavor Chemicals, Eigenverlag, Montclair, N.J., 1969) und deren Maiglöckchen-Note sehr viel weniger ausgeprägt ist als bei dem erfindungsgemäßen 4-Cyclohexyl-2-butanol.

4-Cyclohexyl-2-butanol ist als chemische Verbindung bekannt. In Beilsteins Handbuch der Organischen Chemie Bd. 6, 1. Ergänzungswerk, 4. Aufl. 1931, S. 18, findet sich allerdings nur der Hinweis, dass 4-Cyclohexyl-2-butanol eine "angenehm riechende Flüssigkeit" sei. Eine genauere Beschreibung der geruchlich-sensorischen Eigenschaften fehlt. Insbesondere findet sich kein Hinweis darauf, dass 4-Cyclohexyl-2-butanol einen natürlichen und reintönigen Maiglöckchengeruch besitzt und deshalb geeignet ist, anstelle der bislang üblichen Substanzen als Maiglöckchen-Riechstoff in Parfümkompositionen verwendet zu werden.

Patentschrift US 2447050 beschreibt 4-Cyclohexyl-2-butanol als angenehm riechende Flüssigkeit.

4-Cyclohexyl-2-butanol besitzt einen typischen blumigen Geruch nach Mai glöckchen mit nur einer leichten linaloolartigen Note und mit frischen und fruchtigen Aspekten. Aufgrund dieser sensorisch-geruchlichen Eigenschaften eignet sich 4-Cyclohexyl-2-butanol hervorragend für die Verwendung als Riechstoff in Parfümöl-Kompositionen, die insbesondere für den Einsatz in Waschmitteln und Weichspülern vorgesehen sein können. Bereits in geringen Dosierungen lassen sich mit 4-Cyclohexyl-2-butanol in den resultierenden Kompositionen Effekte einer deutlichen Natürlichkeit erzielen, wobei der geruchliche Gesamteindruck insgesamt verstärkt und harmonisiert und die Ausstrahlung und Diffusivität wahrnehmbar erhöht werden. Insbesondere kann 4-Cyclohexyl-2-butanol in Riechstoffkompositionen zur Erzielung einer klaren, sauberen, transparenten und frischen Duftnote eingesetzt werden. Als Komponente mit ausgesprochenem Maiglöckchengeruch ist 4-Cyclohexyl-2-butanol besonders dafür geeignet, Riechstoffe wie 3-(4t-Butylphenyl)-2-methylpropanal, 3,7-Dimethyl-7-hydroxyoctanal, und 4-(4-Hydroxy-4-methylpentyl)-3-cyclohexencarboxaldehyd, die negative toxikologische Eigenschaften haben, in Parfümkompositionen ganz oder zumindest teilweise zu ersetzen.

4-Cyclohexyl-2-butanol kann in einer Vielzahl von Produkten verwendet werden; es kann als Einzelriechstoff eingesetzt werden, besonders vorteilhaft lässt es sich aber mit anderen Riechstoffen in verschiedenen, unterschiedlichen Mengenverhältnissen zu Riechstoffmischungen kombinieren, wobei auch neuartige und originelle Parfümkompositionen kreiert werden können. Dementsprechend betrifft ein Aspekt der Erfindung auch eine Mischung aus zwei oder mehr Riechstoffen (Riechstoffmischung) und ggf. weiteren Bestandteilen (Lösungsmitteln o.dgl.), welche 4-Cyclohexyl-2-butanol enthält. Vorteilhafterweise enthält die Riechstoffmischung dabei 4-Cyclohexyl-2-butanol in einer Menge, die
(a) die geruchlichen Eigenschaften einer Mischung der sonstigen Bestandteile der Riechstoffmischung in Richtung klarer, sauberer, transparenter und frischer modifiziert und/oder
(b) den geruchlichen Eigenschaften einer Mischung der sonstigen Bestandteile der Riechstoffmischung eine Maiglöckennote hinzufügt.

Beispiele für Riechstoffe, mit denen 4-Cyclohexyl-2-butanol vorteilhaft kombiniert werden kann, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 4rd. Ed., Wiley-VCH, Weinheim 2001.

Im Einzelnen seien genannt:

**Extrakte aus natürlichen Rohstoffen wie Etherische Öle, Concretes, Absolues, Resine, Resinoide, Balsame, Tinkturen wie z. B.**
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucatyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur, Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;

**Einzel-Riechstoffe aus der Gruppe**
**der Kohlenwasserstoffe, wie z. B.** 3-Caren; a-Pinen; b-Pinen; a-Terpinen; g-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
**der aliphatischen Alkohole wie z. B.** Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
**der aliphatischen Aldehyde und deren Acetale wie z. B.** Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd;1-(1-Methoxypropoxy)-(E/Z)-3-hexen;
**der aliphatischen Ketone und deren Oxime wie z. B.** 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
**der aliphatischen schwefelhaltigen Verbindungen wie z. B.** 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
**der aliphatischen Nitrile wie z.B.** 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
**der Ester von aliphatischen Carbonsäuren wie z.B.** (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentylcrotonat;
**der acyclischen Terpenalkohole wie z. B.** Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
**der acyclischen Terpenaldehyde und -ketone wie z. B.** Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
**der cyclischen Terpenalkohole wie z. B.** Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
**der cyclischen Terpenaldehyde und -ketone wie z. B.** Menthon; Isomenthon ; 8-Mercaptomenthan-3-on ; Carvon; Campher; Fenchon; alphalonon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
**der cyclischen Alkohole wie z.B.** 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
**der cycloaliphatischen Alkohole wie z.B.** alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
**der cyclischen und cycloaliphatischen Ether wie z.B.** Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
**der cyclischen und makrocyclischen Ketone wie z.B.** 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
**der cycloaliphatischen Aldehyde wie z.B.** 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
**der cycloaliphatischen Ketone wie z. B.** 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
**der Ester cyclischer Alkohole wie z.B.** 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
**der Ester cycloaliphatischer Alkohole wie z.B.** 1-Cyclohexylethylcrotonat;; der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
**der araliphatischen Alkohole wie z.B.** Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
**der Ester von araliphatischen Alkoholen und aliphatischen Carbonsäuren wie z.B.** Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
**der araliphatischen Ether wie z.B.** 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
**der aromatischen und araliphatischen Aldehyde wie z. B.** Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
**der aromatischen und araliphatischen Ketone wie z.B.** Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
**der aromatischen und araliphatischen Carbonsäuren und deren Ester wie z.B.** Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
**der stickstoffhaltigen aromatischen Verbindungen wie z.B.** 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiffsche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
**der Phenole, Phenylether und Phenylester wie z.B.** Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
**der heterocyclischen Verbindungen wie z.B.** 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
**der Lactone wie z.B.** 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis-und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

In Parfümöl-Kompositionen (=Riechstoffmischungen) liegt die eingesetzte Menge von 4-Cyclohexyl-2-butanol üblicherweise im Bereich von 0,01 bis 99,9 Gew.-%, vorzugsweise 0,01 bis 90 Gew.-%, weiter vorzugsweise 0,1 bis 70 Gew.-% und besonders bevorzugt 1 bis 40 Gew.-%, bezogen auf die gesamte Parfümöl-Komposition.

4-Cyclohexyl-2-butanol enthaltende Parfümöl-Kompositionen werden vorteilhaft in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Ein weiterer Aspekt der Erfindung betrifft parfümierte Produkte, die eine erfindungsgemäße Riechstoffmischung enthalten. Bei einem derartigen parfümierten Produkt kann es sich insbesondere um ein alkoholisches Parfüm, ein Körperpflegeprodukt oder ein im Haushalt zu verwendendes Reinigungs- oder Pflegeprodukt handeln.

Bei den Körperpflegeprodukten kann es sich insbesondere um Seifen, Shampoos, Duschgele, Badezusätze, Deodorantien, Hautcremes oder Körperlotionen handeln.

Bei den im Haushalt zu verwendenden Reinigungs- und Pflegeprodukten kann es sich insbesondere um Waschmittel, Wäscheweichspüler, Reiniger und Raumluftverbesserer handeln. Hinsichtlich des Einsatzes von Maiglöckchen-Riechstoffen in Waschmitteln, Wäscheweichspülern u.dgl. wird auf die obigen Ausführungen Bezug genommen. 4-Cyclohexyl-2-butanol hat sich insoweit als ein Riechstoff von herausragender Qualität erwiesen.

Ganz allgemein wird ein erfindungsgemäßes parfümiertes Produkt hergestellt, indem (a) 4-Cyclohexyl-2-butanol oder (b) eine erfindungsgemäße Riechstoffmischung (siehe dazu oben), ggf. gemeinsam mit weiteren Parfümbestandteilen, dem (zu parfümierenden) Produkt zugesetzt wird, so dass das resultierende parfümierte Produkt aufgrund der Anwesenheit von 4-Cyclohexyl-2-butanol den vorteilhaften, also insbesondere natürlichen, blumigen und/oder maiglöckchenartigen Geruch vermittelt.

Dementsprechend betrifft die vorliegende Erfindung gemäß einem weiteren Aspekt auch ein Verfahren zum Vermitteln eines Maiglöckchen-Geruchs oder einer Maiglöckchen-Geruchsnote und/oder zum Modifizieren eines vorhandenen Geruches in Richtung klarer, sauberer, transparenter und frischer, wobei einer Substanz (einem in sensorischer Hinsicht zu modifizierenden Vorprodukt) eine sensorisch wirksame Menge von 4-Cyclohexyl-2-butanol zugesetzt wird.

Für manche Anwendungen ist es vorteilhaft, 4-Cyclohexyl-2-butanol enthaltende Parfümöle an einem Trägerstoff adsorbiert einzusetzen, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer; Cellulosebasierende Stoffe, Zucker oder Kunststoffe wie PVC, Polyvinylacetate oder Polyurethane sein.

Für andere Anwendungen ist es vorteilhaft, 4-Cyclohexyl-2-butanol enthaltende Parfümöle mikroverkapselt, sprühgetrocknet, als Einschluß-Komplex oder als Extrusions-Produkt einzusetzen und in dieser Form dem zu parfümierenden (Vor-)Produkt hinzufügen.

Die Eigenschaften derart modifizierter Parfümöle werden in manchen Fällen durch sogenanntes "Coaten" mit geeigneten Materialien im Hinblick auf eine gezieltere Duftfreisetzung weiter optimiert, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

4-Cyclohexyl-2-butanol enthaltende Parfümöle können in konzentrierter Form, in Lösungen oder in oben beschriebener modifizierter Form verwendet werden für die Herstellung insbesondere von flüssigen Waschmitteln, pulverförmigen Waschmitteln, Wäschevorbehandlungsmitteln wie Bleichmittel, Einweichmittel und Fleckenentfernern, Wäscheweichspülern, Waschseifen und Waschtabletten (siehe die obigen Ausführungen), oder von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, Desinfektionsmitteln, Oberflächendesinfektionsmittein sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-öl-in-Wasser-Typ wie z.B. Hautcremes- und -lotionen, Gesichtscremes und -lotionen, Sonnenschutzcremes-und -lotionen, After-sun-cremes und-lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Die Herstellung von 4-Cyclohexyl-2-butanol kann auf an sich bekannte Weise durch Hydrierung von Benzalaceton (4-Phenyl-3-buten-2-on) erfolgen. Zweckmäßigerweise erfolgt die Hydrierung in einem Schritt, wobei die Ketogruppe, die Doppelbindung und der aromatische Ring hydriert werden. Ein geeigneter Hydrierkatalysator ist z.B. Platin (Beilsteins Handbuch der Organischen Chemie Bd. 6, 1. Ergänzungswerk, 4. Aufl. 1931, S. 18; 2. Ergänzungswerk, 1944, S. 38). Für eine technische Durchführung sind Katalysatoren wie Raney-Nickel (Beilsteins Handbuch der Organischen Chemie Bd. 6, 3. Ergänzungswerk, 1965, S. 123) und Ruthenium auf Kohle (Houben-Weyl, Methoden der Organischen Chemie Bd. 4/1c Ss. 20-21, 185) vorteilhaft. Handelsübliche Qualitäten der letztgenannten Katalysatoren führen zu ausgezeichneten Ergebnissen, in Bezug auf Ausbeute und sensorische Qualität, wenn bei den durchgeführten Hydrierungen das Gewichtsverhältnis von eingesetztem Katalysator zum 4-Phenyl-3-buten-2-on 0,001 bis 0,1 : 1, bevorzugt 0,005 bis 0,05 : 1, beträgt und während der Hydrierung der Wasserstoffdruck zwischen 5 und 100 bar, bevorzugt zwischen 10 und 50 bar, und die Temperatur zwischen 80 und 200°C, bevorzugt zwischen 100 und 150°C, liegt.

Die folgenden Beispiele erläutern die Erfindung:

### Beispiel 1

### Herstellung von 4-Cyclohexyl-2-butanol

1022 g (7 mol) Benzalaceton werden in einem 2l-Stahlautoklaven in Gegenwart von 10 g eines Ruthenium-Katalysators (5 Gew.-% Ru auf Aktivkohle, Wassergehalt des Katalysators ca. 50 Gew.-%) bei einer Temperatur von 130°C und einem Druck von 20 bar solange hydriert, bis die Wasserstoffaufnahme beendet ist; die Reaktionsdauer lag typischerweise bei 6 bis 8 h. Nach Abfiltrieren des Katalysators wird über einer 30-cm-Füllkorperkolonne fraktioniert. Bei einem Sdp. von 85°C bei 4 mbar werden 995 g (91 % d. Th.) analytisch und sensorisch reines 4-Cyclohexyl-2-butanol erhalten.

### Beispiel 2

### Herstellung eines Parfümöles mit Maiglöckchen-Geruch unter Verwendung von 4-Cyclohexyl-2-butanol

Es werden zunächst folgende Komponenten vermischt:

| Komponente | Gewichtsteile |
|---|---|
| Methyldihydrojasmonat | 60 |
| Diethylphthalat | 71 |
| Indol | 1 |
| Geraniol | 25 |
| Phenylethylalkohol | 50 |
| I-Citronellol | 60 |
| Hexylzimtaldehyd | 275 |
| cis-/trans-3-Hexenylacetat | 3 |
| Linalool | 45 |
| Linalylacetat | 10 |
| Summe: | 600 |

Durch Hinzufügen von 300 Gewichtsteilen 4-Cyclohexyl-2-butanol erhält die Parfümöl-Komposition einen typischen natürlichen Maiglöckchen-Geruch.

Vergleicht man die so erhaltene Parfümkomposition mit einer, der statt des 4-Cyclohexyl-2-butanols ein gleicher Anteil an 3-(4t-Butylphenyl)-2-methylpropanal oder 4-(4-Hydroxy-4-methylpentyl)-3-cyclohexencarboxaldehyd (also ein gleicher Anteil eines üblichen Maiglöckchen-Riechstoffs) zugesetzt wurde, so zeigt sich, dass die Komposition mit 4-Cyclohexyl-2-butanol bei ansonsten ähnlichem Geruchseindruck wesentlich kräftiger und ausgiebiger ist.

### Beispiel 3

### Herstellung eines Parfümöles mit einem blumig-fruchtigem Geruch unter Verwendung von 4-Cyclohexyl-2-butanol

Es werden folgende Komponenten vermischt:

| Komponente | Gewichtsteile |
|---|---|
| Methyldihydrojasmonat | 180 |
| Diethylphthalat | 50,7 |
| beta-Damascon | 0,3 |
| Helional IFF | 15 |
| Geraniol | 60 |
| Ylang-Ylang-Öl III | 15 |
| Phenylethylalkohol | 90 |
| Vanillin | 10 |
| gamma-Methylionon | 15 |
| Iso E Super IFF | 225 |
| Muscon | 4 |
| Benzylacetat | 30 |
| 1,15-Pentadecanolid | 185 |
| Bergamotte-Öl afrikan. künstl. | 60 |
| Summe: | 940 |

Durch Hinzufügen von 60 Gewichtsteilen 4-Cyclohexyl-2-butanol wird die Parfümöl-Komposition harmonischer und voluminöser, die bereits vorhandene komplex-blumige Note verstärkt und aufgrund des hinzutretenden Maiglöckchen-Aspekts wesentlich ästhetischer.

## Patentansprüche

1. Verwendung von 4-Cyclohexyl-2-butanol als Maiglöckchen-Riechstoff.

2. Verwendung von 4-Cyclohexyl-2-butanol in Riechstoffkompositionen zur Erzielung einer klaren, sauberen, transparenten und frischen Duftnote.

3. Parfümiertes Produkt, enthaltend eine Mischung aus zwei oder mehr Riechstoffen und gegebenenfalls weiteren Bestandteilen, **dadurch gekennzeichnet, dass** die Mischung 4-Cyclohexyl-2-butanol
in einer Menge enthält, die
(a) die geruchlichen Eigenschaften einer Mischung der sonstigen Bestandteile der Riechstoffmischung in Richtung klarer, sauberer, transparenter und frischer modifiziert und/oder
(b) den geruchlichen Eigenschaften einer Mischung der sonstigen Bestandteile der Riechstoffmischung eine Maiglöckchennote hinzufügt, und
wobei das Produkt ein alkoholisches Parfüm, ein Körperpflegeprodukt oder ein im Haushalt zu verwendendes Reinigungs- oder Pflegeprodukt ist.

4. Verfahren zur Herstellung eines parfümierten Produkts gemäß Anspruch 3, **dadurch gekennzeichnet, dass** (a) 4-Cyclohexyl-2-butanol oder (b) eine Mischung mit zwei oder mehr Riechstoffen enthaltend 4-Cyclohexyl-2-butanol, gegebenenfalls gemeinsam mit weiteren Parfümbestandteilen, dem Produkt zugesetzt wird, so dass das parfümierte Produkt aufgrund der Anwesenheit von 4-Cyclohexyl-2-butanol einen natürlichen, blumigen und/oder maiglöckchenartigen Geruch vermittelt

5. Verwendung von 4-Cyclohexyl-2-butanol als Riechstoff in einem Körperpflegeprodukt oder einem im Haushalt zu verwendenden Reinigungs- oder Pflegeprodukt.

6. Verfahren zum Vermitteln eines Maiglöckchen-Geruchs und/oder einer Maiglöckchen-Geruchsnote oder zum Modifizieren eines vorhandenen Geruches in Richtung klarer, sauberer, transparenter und frischer, wobei einer Substanz eine sensorisch wirksame Menge von 4-Cyclohexyl-2-butanol zugesetzt wird.

## Claims

1. Use of 4-cyclohexyl-2-butanol as a lily of the valley fragrance substance.

2. Use of 4-cyclohexyl-2-butanol in fragrance substance compositions to obtain a clear, clean, transparent and fresh fragrance note.

3. Perfumed product containing a blend of two or more fragrance substances and optionally other constituents, **characterised in that** the blend contains 4-cyclohexyl-2-butanol in a quantity which
(a) modifies the olfactory properties of a blend of the other constituents of the fragrance substance blend to make it clearer, cleaner, more transparent and fresher and/or
(b) adds a lily of the valley note to the olfactory properties of a blend of the other constituents of the fragrance substance blend, and
wherein the product is an alcoholic perfume, a personal hygiene product or a cleaning or care product for use in the home.

4. Process for producing a perfumed product according to claim 3, **characterised in that** (a) 4-cyclohexyl-2-butanol or (b) a blend with two or more fragrance substances containing 4-cyclohexyl-2-butanol, optionally together with other perfume constituents, is added to the product such that the perfumed product gives off a natural, floral and/or lily of the valley-like fragrance owing to the presence of 4-cyclohexyl-2-butanol.

5. Use of 4-cyclohexyl-2-butanol as a fragrance substance in a personal hygiene product or a cleaning or care product for use in the home.

6. Process for imparting a lily of the valley fragrance and/or a lily of the valley fragrance note or for modifying an existing fragrance to make it clearer, cleaner, more transparent and fresher, wherein a sensorially effective amount of 4-cyclohexyl-2-butanol is added to a substance.

## Revendications

1. Utilisation de 4-cyclohexyl-2-butanol en tant que parfum de muguet.

2. Utilisation de 4-cyclohexyl-2-butanol dans des compositions de parfums dans le but d'obtenir une note parfumée claire, propre, transparente et fraîche.

3. Produit parfumé contenant un mélange de deux ou plusieurs parfums et le cas échéant d'autres constituants, **caractérisé en ce que** le mélange contient du 4-cyclohexyl-2-butanol en une quantité qui
(a) modifie les propriétés odorantes d'un mélange des autres constituants du mélange de parfums dans le sens d'une note claire, propre, transparente et fraîche, et/ou
(b) ajoute une note de muguet aux propriétés odorantes d'un mélange des autres constituants du mélange de parfums, et
le produit étant un parfum à base alcoolique, un produit d'hygiène corporelle ou un produit de nettoyage ou d'entretien à usage ménager.

4. Procédé de production d'un produit parfumé selon la revendication 3, **caractérisé en ce que** l'on ajoute au produit (a) du 4-cyclohexyl-2-butanol ou (b) un mélange de deux ou plusieurs parfums contenant du 4-cyclohexyl-2-butanol, le cas échéant en même temps que d'autres constituants parfumés, afin que le produit parfumé dégage un parfum naturel, floral et/ou de muguet grâce à la présence du 4-cyclohexyl-2-butanol.

5. Utilisation de 4-cyclohexyl-2-butanol comme parfum dans un produit d'hygiène corporelle ou dans un produit de nettoyage ou d'entretien à usage ménager.

6. Procédé permettant de conférer un parfum de muguet et/ou une note parfumée de muguet ou de modifier une odeur existante dans le sens d'une note claire, propre, transparente et fraîche, dans lequel une quantité de 4-cyclohexyl-2-butanol efficace au plan sensoriel est ajoutée à une substance.
